(19) Europäisches Patentamt
European Patent Office
Office européen des brevets

(11) Veröffentlichungsnummer: **0 401 256 B1**

# (12) EUROPÄISCHE PATENTSCHRIFT

(45) Veröffentlichungstag der Patentschrift: **26.05.93**

(51) Int. Cl.$^5$: **A61K 31/445**

(21) Anmeldenummer: **89902511.8**

(22) Anmeldetag: **16.02.89**

(86) Internationale Anmeldenummer:
**PCT/EP89/00140**

(87) Internationale Veröffentlichungsnummer:
**WO 89/07443 (24.08.89 89/20)**

## (54) OPTISCH REINES DEXNIGULDIPIN UND DESSEN DERIVATE ZUR BEHANDLUNG VON TUMORERKRANKUNGEN.

(30) Priorität: **19.02.88 CH 630/88**
**19.02.88 CH 629/88**

(43) Veröffentlichungstag der Anmeldung:
**12.12.90 Patentblatt 90/50**

(45) Bekanntmachung des Hinweises auf die Patenterteilung:
**26.05.93 Patentblatt 93/21**

(84) Benannte Vertragsstaaten:
**AT BE CH DE FR GB IT LI LU NL SE**

(56) Entgegenhaltungen:
**EP-A- 0 123 850**
**EP-A- 0 176 956**
**EP-A- 0 240 828**
**EP-A- 0 242 829**
**DE-A- 3 709 796**

**J. CARDIOVASC. PHARMACOL., Band 10, Nr. 3, 1987, Raven Press Ltd., New York, NY (US); G.FISCHER et al., Seiten 268-273***

(73) Patentinhaber: **Byk Gulden Lomberg Chemische Fabrik GmbH**
**Byk-Gulden-Strasse 2**
**W-7750 Konstanz(DE)**

(72) Erfinder: **KLEMM, Kurt**
**Im Weinberg 2**
**W-7753 Allensbach(DE)**
Erfinder: **ULRICH, Wolf-Rüdiger**
**Hebelstr. 3**
**W-7750 Konstanz(DE)**
Erfinder: **FLOCKERZI, Dieter**
**Ackerweg 26**
**W-7753 Allensbach(DE)**
Erfinder: **SANDERS, Karl**
**Felchengang 23**
**W-7750 Konstanz(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

Erfinder: **BELLER, Klaus-Dieter**
**Franz Moser-Str. 5**
**W-7750 Konstanz 26(DE)**
Erfinder: **SCHUDT, Christian**
**Hoheneggstr. 102**
**W-7750 Konstanz(DE)**
Erfinder: **BOER, Rainer**
**Löhrystr. 4**
**W-7750 Konstanz(DE)**
Erfinder: **GIETZEN, Klaus, Abt. Pharmakologie u. Toxikologie**
**Univ. Ulm, Oberer Eselsberg**
**W-7900 Ulm(DE)**

## Beschreibung

Die Erfindung betrifft optisch reine Diarylverbindungen mit antineoplastischer Aktivität, ihre therapeuti-sche Anwendung und Arzneimittel, die sie entalten. Die Verbindungen werden in der pharmazeutischen Industrie zur Herstellung von Arzneimitteln eingesetzt.

### Bekannter technischer Hintergrund

Die Verwendung von 1,4-Dihydropyridinen mit calciumkanal-blockierender Aktivität zur Verringerung der Metastasenbildung und des Tumorwachstums bei Säugern wird in der europäischen Patentanmeldung 0 123 850 beschrieben. Die antineoplastische Wirkung der dort untersuchten 1,4-Dihydropyridine (wie z.B. Nimodipin oder Nifedipin) wird deren calciumkanal-blockierenden Aktivität zugeschrieben, die ihren Ein-satz in der Humanmedizin zur Behandlung von Herz- und Gefäßerkrankungen gestattet. - In der europäischen Patentanmeldung 0 176 956 werden 1,4-Dihydropyridine mit Diarylpiperidylesterrest und ihre Anwendung bei der Behandlung cardiovaskulärer Erkrankungen offenbart. - In der europäischen Patentanmeldung 0 240 828 wird ein bestimmtes Enantiomer, der (+)-1,4-Dihydro-2,6-dimethyl-4-(3-nitrophenyl)-pyridin-3,5-dicarbonsäure-3-methyl-5-[3-(4,4-diphenyl-1-piperidinyl)-propyl]-ester und seine Verwendung für die Behandlung cardiovaskulärer Erkrankungen beansprucht. - In der europäischen Patentanmeldung 0 242 829 werden 2-Amino-1,4-dihydropyridine mit Diarylpiperidy-lesterrest und ihre Anwendung bei der Behandlung cardiovaskulärer Erkrankungen offenbart.

### Beschreibung der Erfindung

Gegenstand der Erfindung ist die Verwendung von optisch reinen Diarylverbindungen der Formel I

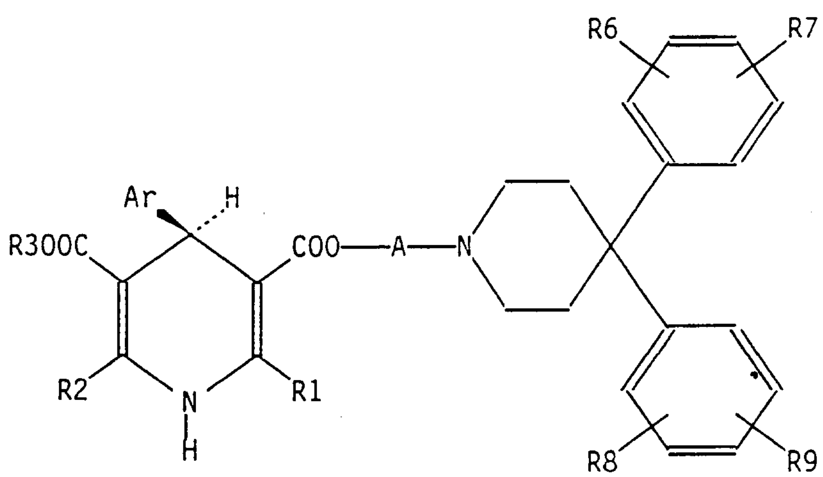

(I)

worin

Ar einen Cyclus der Formel

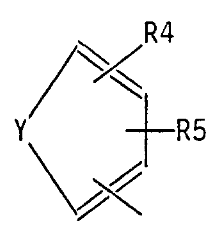

darstellt, in dem Y Sauerstoff (O), Schwefel (S), Vinylen (-CH=CH-), Azomethin (-CH=N-) oder eine Gruppe der Formel

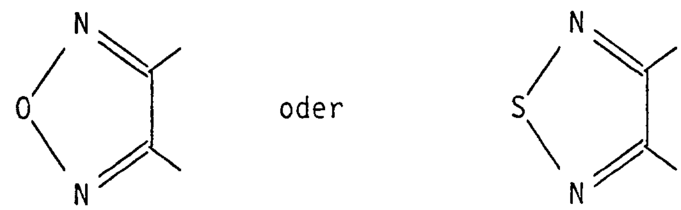

bedeutet,

| | |
|---|---|
| R1 | Wasserstoff, 1 – 6C – Alkyl oder 3 – 7C – Alkoxyalkyl bedeutet, |
| R2 | Wasserstoff, Amino ($NH_2$), 1 – 6C – Alkyl oder 3 – 7C – Alkoxyalkyl bedeutet, |
| R3 | Wasserstoff, 1 – 6C – Alkyl oder 3 – 7C – Alkoxyalkyl bedeutet, |
| R4 und R5 | gleich oder verschieden sind und Wasserstoff, Hydroxy, Halogen, Nitro, Cyano, Trifluormethyl, 1 – 4C – Alkyl, 1 – 4C – Alkoxy, ganz oder teilweise durch Fluor substituiertes 1 – 4C – Alkoxy, 1 – 4C – Alkoxycarbonyl, 2 – 5C – Acyl, Amino, Mono – oder Di – 1 – 4C – alkylamino oder gemeinsam Methylendioxy bedeuten, |
| R6, R7, R8 und R9 | gleich oder verschieden sind und Wasserstoff, Hydroxy, Halogen, 1 – 4C – Alkyl, 1 – 4C – Alkoxy oder ganz oder teilweise durch Fluor substituiertes 1 – 4C – Alkoxy bedeuten, |
| A | 2 – 5C – Alkylen oder A1 – O – A2 bedeutet, wobei A1 2 – 4C – Alkylen und A2 2 – 4C – Alkylen oder 2C – Alkylenoxy – 2C – alkylen bedeutet, |

und ihren Salzen zur Herstellung von Arzneimitteln für die Behandlung von Tumorerkrankungen.

Die Anordnung der Substituenten Ar und H in 4 – Position des 1,4 – Dihydropyridinringes wurde aufgrund der Veröffentlichung von K. Tamazawa et al., J. Med. Chem. 29, 2504 (1986) vorgenommen. Alternativ können die Verbindungen I auch als optisch reine, konfigurativ einheitliche Verbindungen der Formel Ia,

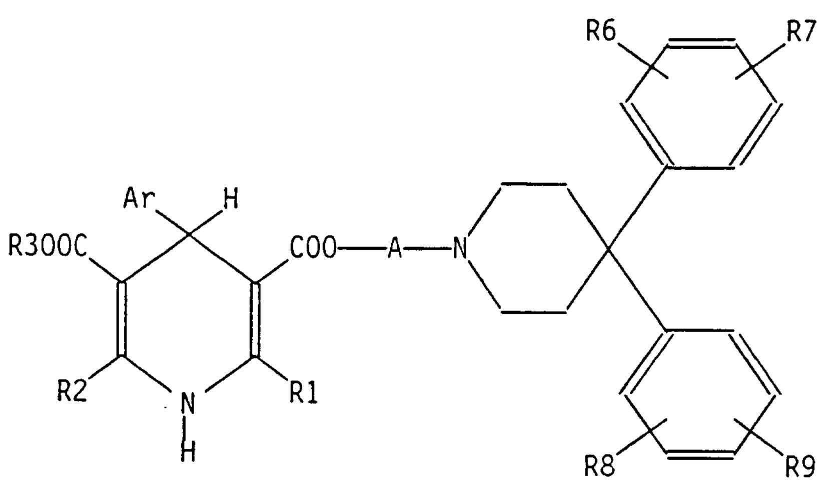

(Ia)

bezeichnet werden, in denen Ar, R1, R2, R3, R4, R5, R6, R7, R8, R9 und A die oben angegebenen Bedeutungen haben, und die in der 4 – Position des Dihydropyridins die gleiche Konfiguration aufweisen wie das als Vorprodukt eingesetzte diastereomere ( + ) – 1 – Ethoxymethyl – 1,4 – dihydro – 5 – methoxycarbonyl – 2,6 – dimethyl – 4 – (3 – nitrophenyl) – pyridin – 3 – carbonsäure – Cinchoninsalz, das das linear polarisierte Licht der Wellenlänge 589 nm mit $[\alpha]_D^{22}$ = + 101,5° (c = 1, Chloroform) dreht.

1 – 6C – Alkyl ist geradkettig oder verzweigt und bedeutet beispielsweise einen Hexyl – , Neopentyl – , Isopentyl – , Butyl – , i – Butyl – , sec. – Butyl, t – Butyl – , Propyl – , Isopropyl – oder insbesondere Ethyl – oder Methylrest.

3 – 7C – Alkoxyalkyl steht beispielsweise für einen Methoxyethyl – , Ethoxyethyl – , Propoxyethyl – , Isopropoxyethyl – , Butoxyethyl – , Methoxypropyl – , 2 – Methoxy – 1 – methylethyl – oder 2 – Ethoxy – 1 – methylethylrest.

Halogen im Sinne der Erfindung bedeutet Brom und insbesondere Fluor und Chlor.

1-4C-Alkyl ist geradkettig oder verzweigt und bedeutet beispielsweise einen Butyl-, i-Butyl, sec.-Butyl-, t-Butyl-, Propyl-, Isopropyl-, Ethyl- oder insbesondere Methylrest.

1-4C-Alkoxy enthält neben dem Sauerstoffatom einen der vorstehend genannten 1-4C-Alkylreste. Bevorzugt ist der Methoxyrest.

Ganz oder teilweise durch Fluor substituiertes 1-4C-Alkoxy ist beispielsweise 1,1,2,2-Tetrafluorethoxy, Trifluormethoxy, 2,2,2-Trifluorethoxy oder Difluormethoxy.

1-4C-Alkoxycarbonyl enthält neben der Carbonylgruppe einen der vorstehend genannten 1-4C-Alkoxyreste. Bevorzugt sind der Methoxycarbonyl- und der Ethoxycarbonylrest.

2-5C-Acyl enthält neben der Carbonylgruppe einen der vorstehend genannten 1-4C-Alkylreste. Bevorzugt ist der Acetylrest.

Mono- oder Di-1-4C-alkylamino enthält neben dem Stickstoffatom einen oder zwei der vorstehend genannten 1-4C-Alkylreste Bevorzugt ist Di-1-4C-alkylamino, und hier insbesondere Dimethyl-, Diethyl- oder Diisopropylamino.

2-5C-Alkylen ist beispielsweise Tetramethylen, 1,2-Dimethylethylen, 1,1-Dimethylethylen, 2,2-Dimethylethylen, Isopropyliden, 1-Methylethylen, 2-Ethylpropylen und insbesondere Ethylen oder Propylen (Trimethylen).

2-4C-Alkylen steht für Ethylen ($-CH_2-CH_2-$), Trimethylen ($-CH_2-CH_2-CH_2-$) und Tetramethylen ($-CH_2-CH_2-CH_2-CH_2-$), wobei Ethylen bevorzugt ist.

2-C-Alkylenoxy-2C-alkylen steht für Ethylen, das durch Ethylenoxy substituiert ist ($-CH_2-CH_2-O-CH_2-CH_2-$).

Als Salze kommen alle Salze mit Säuren in Betracht. Besonders erwähnt seien die pharmakologisch verträglichen Salze der in der pharmazeutischen Industrie üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Metembonat, Stearat, Tosilat, 2-Hydroxy-3-naphthoat, 3-Hydroxy-2-naphthoat oder Mesilat.

Hervorzuheben ist die erfindungsgemäße Verwendung von Verbindungen der Formel I, worin Ar Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2,3-Methylendioxyphenyl, 2-Trifluormethylphenyl, 3-Trifluormethylphenyl oder 2,1,3-Benzoxdiazol-4-yl, R1 Methyl, R2 Amino oder Methyl, R3 Methyl, Ethyl oder Methoxyethyl, R6 Wasserstoff, R7 Wasserstoff oder Methoxy, R8 Wasserstoff, R9 Wasserstoff oder Methoxy, A Ethylen, Propylen, Butylen, 1,1-Dimethylethylen, 2,2-Dimethylethylen oder A1-O-A2 bedeutet, wobei A1 Ethylen und A2 Ethylen oder Ethylenoxyethylen bedeuten, und ihren Salzen.

Besonders hervorzuheben ist einerseits die erfindungsgemäße Verwendung von Verbindungen der Formel I, worin Ar 3-Nitrophenyl, 2-Chlorphenyl, 2,3-Dichlorphenyl, 2-Trifluormethylphenyl, 2-Difluormethoxyphenyl, 2,3-Methylendioxyphenyl oder 2,1,3-Benzoxdiazol-4-yl, R1 Methyl, R2 Methyl, R3 Methyl, Ethyl oder Methoxyethyl, R6, R7, R8 und R9 Wasserstoff und A Ethylen oder Propylen bedeutet, und ihren Salzen.

Besonders hervorzuheben ist andererseits die erfindungsgemäße Verwendung von Verbindungen der Formel I, worin Ar 3-Nitrophenyl, 2-Chlorphenyl, 2,3-Dichlorphenyl, 2-Trifluormethylphenyl, 2-Difluormethoxyphenyl, 2,3-Methylendioxyphenyl oder 2,1,3-Benzoxdiazol-4-yl, R1 Methyl, R2 Amino, R3 Methyl, Ethyl oder Methoxyethyl, R6, R7, R8 und R9 Wasserstoff und A Ethylen oder Propylen bedeutet, und ihren Salzen.

Besonders hervorzuheben ist außerdem die erfindungsgemäße Verwendung von Verbindungen der Formel I, worin Ar 3-Nitrophenyl, 2-Chlorphenyl, 2,3-Dichlorphenyl, 2-Trifluormethylphenyl, 2-Difluormethoxyphenyl, 2,3-Methylendioxyphenyl oder 2,1,3-Benzoxdiazol-4-yl, R1 Methyl, R2 Methyl, R3 Methyl, Ethyl oder Methoxyethyl, R6, R7, R8 und R9 Wasserstoff und A A1-O-A2 bedeutet, wobei A1 Ethylen und A2 Ethylen oder Ethylenoxyethylen bedeutet, und ihren Salzen.

Bevorzugt ist einerseits die erfindungsgemäße Verwendung von Verbindungen der Formel I, worin Ar 3-Nitrophenyl oder 2,3-Dichlorphenyl, R1 Methyl, R2 Methyl, R3 Methyl oder Ethyl, R6, R7, R8 und R9 Wasserstoff und A Ethylen oder Propylen bedeutet, und ihren Salzen.

Bevorzugt ist andererseits die erfindungsgemäße Verwendung von Verbindungen der Formel I, worin Ar 3 – Nitrophenyl oder 2,3 – Dichlorphenyl, R1 Methyl, R2 Amino, R3 Methyl oder Ethyl, R6, R7, R8 und R9 Wasserstoff und A Ethylen oder Propylen bedeutet, und ihren Salzen.

Bevorzugt ist außerdem die erfindungsgemäße Verwendung von Verbindungen der Formel I, worin Ar 3 – Nitrophenyl oder 2,3 – Dichlorphenyl, R1 Methyl, R2 Methyl, R3 Methyl oder Ethyl, R6, R7, R8 und R9 Wasserstoff und A A1 – O – A2 bedeutet, wobei A1 Ethylen und A2 Ethylen bedeutet, und ihren Salzen.

Hervorzuheben ist beispielsweise die erfindungsgemäße Verwendung folgender Verbindungen:
(R) – 1,4 – Dihydro – 2,6 – dimethyl – 4 – (3 – nitrophenyl) – pyridin – 3,5 – dicarbonsäure – 3 – methyl – 5 – [2 – (4,4 – diphenyl – 1 – piperidinyl) – ethyl] – ester,
(R) – 4 – (3 – Cyanphenyl) – 1,4 – dihydro – 2,6 – dimethylpyridin – 3,5 – dicarbonsäure – 3 – methyl – 5 – [3 – (4,4 – diphenyl – 1 – piperidinyl) – propyl] – ester,
(R) – 1,4 – Dihydro – 2,6 – dimethyl – 4 – (3 – nitrophenyl) – pyridin – 3,5 – dicarbonsäure – 3 – methyl – 5 – [2 – (4,4 – diphenol – 1 – piperdinyl) – 2 – methyl – propyl] – ester,
(R) – 1,4 – Dihydro – 2,6 – dimethyl – 4 – (3 – nitrophenyl) – pyridin – 3,5 – dicarbonsäure – 3 – ethyl – 5 – [3 – (4,4 – diphenyl – 1 – piperidinyl) – propyl] – ester
(R) – 1,4 – Dihydro – 2,6 – dimethyl – 4 – [3 – (1,1,2,2, – tetraflourethoxi) – phenyl] – pyridin – 3,5 – dicarbonsäure – 3 – methyl – 5 – [3 – (4,4 – diphenyl – 1 – piperidinyl) – propyl] – ester,
(R) – 1,4 – Dihydro – 2,6 – dimethyl – 4 – [3 – (1,1,2,2, – tetraflourethoxi) – phenyl] – pyridin – 3,5 – dicarbonsäure – 3 – methyl – 5 – [2 – (4,4 – diphenyl – 1 – piperidinyl) – ethyl] – ester,
(S) – 1,4 – Dihydro – 2,6 – dimethyl – 4 – (3 – nitrophenyl) – pyridin – 3,5 – dicarbonsäure – 3 – (2 – methoxiethyl) – 5 – [2 – (4,4 – diphenyl – 1 – piperidinyl) – ethyl] – ester,
(S) – 1,4 – Dihydro – 2,6 – dimethyl – 4 – (3 – nitrophenyl) – pyridin – 3,5 – dicarbonsäure – 3 – (2 – methoxiethyl) – 5 – [3 – (4,4 – diphenyl – 1 – piperidinyl) – propyl] – ester,
(R) – 1,4 – Dihydro – 2,6 – dimethyl – 4 – (3 – nitrophenyl) – pyridin – 3,5 – dicarbonsäure – 3 – methyl – 5 – {3 – [4,4 – di – (4 – methoxiphenyl) – 1 – piperidinyl] – propyl} – ester,
(R) – 1,4 – Dihydro – 2,6 – dimethyl – 4 – (3 – nitrophenyl) – pyridin – 3,5 – dicarbonsäure – 3 – methyl – 5 – [4 – (4,4 – diphenyl – 1 – piperidinyl) – butyl] – ester,
(R) – 1,4 – Dihydro – 2,6 – dimethyl – 4 – (3 – nitrophenyl) – pyridin – 3,5 – dicarbonsäure – 3 – methyl – 5 – [1,1 – dimethyl – 2 – (4,4 – diphenyl – 1 – piperidinyl) – ethyl] – ester,
(R) – 1,4 – Dihydro – 2,6 – dimethyl – 4 – (2 – difluormethoxiphenyl) – pyridin – 3,5 – dicarbonsäure – 3 – ethyl – 5 – [3 – (4,4 – diphenyl – 1 – piperidinyl) – propyl] – ester,
(R) – 1,4 – Dihydro – 2,6 – dimethyl – 4 – [3 – (1,1,2,2 – tetrafluorethoxi) – phenyl] – pyridin – 3,5 – dicarbonsäure – 3 – ethyl – 5 – [3 – (4,4 – diphenyl – 1 – piperidinyl) – propyl] – ester,
(R) – 1,4 – Dihydro – 2,6 – dimethyl – 4 – (3 – difluoromethoxiphenyl) – pyridin – 3,5 – dicarbonsäure – 3 – ethyl – 5 – [3 – (4,4 – diphenyl – 1 – piperidinyl) – propyl] – ester,
(R) – 4 – (2,3 – Dichlorphenyl) – 1,4 – dihydro – 2,6 – dimethylpyridin – 3,5 – dicarbonsäure – 3 – methyl – 5 – [3 – (4,4 – diphenyl – 1 – piperidinyl) – propyl] – ester,
(R) – 4 – (2,1,3 – Benzoxdiazol – 4 – yl) – 1,4 – dihydro – 2,6 – dimethyl – pyridin – 3,5 – dicarbonsäure – 3 – methyl – 5 – [3 – (4,4 – diphenyl – 1 – piperidinyl) – propyl] – ester,
(R) – 1,4 – Dihydro – 2,6 – dimethyl – 4 – (3 – fluorphenyl) – pyridin – 3,5 – dicarbonsäure – 3 – methyl – 5 – [3 – (4,4 – diphenyl – 1 – piperidinyl) – propyl] – ester,
(R) – 1,4 – Dihydro – 2,6 – dimethyl – 4 – (2 – trifluormethylphenyl) – pyridin – 3,5 – dicarbonsäure – 3 – methyl – 5 – [3 – (4,4 – diphenyl – 1 – piperidinyl) – propyl] – ester,
(R) – 4 – (2 – Cyanphenyl) – 1,4 – dihydro – 2,6 – dimethylpyridin – 3,5 – dicarbonsäure – 3 – ethyl – 5 – [3 – (4,4 – diphenyl – 1 – piperidinyl) – propyl] – ester,
(R) – 4 – (2 – Chlorphenyl) – 1,4 – dihydro – 2,6 – dimethylpyridin – 3,5 – dicarbonsäure – 3 – methyl – 5 – [3 – (4,4 – diphenyl – 1 – piperidinyl) – propyl] – ester,
(R) – ( + ) – 1,4 – Dihydro – 2,6 – dimethyl – 4 – (3 – nitrophenyl) – pyridin – 3,5 – dicarbonsäure – 3 – methyl – 5 – [3 – (4,4 – diphenyl – 1 – piperidinyl) – propyl] – ester,
(R) – 2 – Amino – 1,4 – dihydro – 6 – methyl – 4 – (3 – nitrophenyl) – pyridin – 3,5 – dicarbonsäure – 3 – ethyl – 5 – [2 – (4,4 – diphenyl – 1 – piperidinyl) – ethyl] – ester,
(S) – 2 – Amino – 1,4 – dihydro – 6 – methyl – 4 – (3 – nitrophenyl) – pyridin – 3,5 – dicarbonsäure – 3 – (2 – methoxyethyl) – 5 – [3 – (4,4 – diphenyl – 1 – piperidinyl) – propyl] – ester,
(R) – 2 – Amino – 1,4 – dihydro – 6 – methyl – 4 – (3 – nitrophenyl) – pyridin – 3,5 – dicarbonsäure – 3 – methyl – 5 – [4 – (4,4 – diphenyl – 1 – piperidinyl] – butyl] – ester,
(R) – 2 – Amino – 1,4 – dihydro – 6 – methyl – 4 – (2 – nitrophenyl) – pyridin – 3,5 – dicarbonsäure – 3 – methyl – 5 – [2 – (4,4 – diphenyl – 1 – piperidinyl) – ethyl] – ester,
(R) – 2 – Amino – 1,4 – dihydro – 6 – ethyl – 4 – (3 – nitrophenyl) – pyridin – 3,5 – dicarbonsäure – 3 – methyl –

5 – [2 – (4,4 – diphenyl – 1 – piperidinyl) – ethyl] – ester,
(R) – 2 – Amino – 1,4 – dihydro – 6 – methyl – 4 – (3 – nitrophenyl) – pyridin – 3,5 – dicarbonsäure – 3 – (propyl – 2) – 5 – [2 – (4,4 – diphenyl – 1 – piperidinyl) – ethyl] – ester,
(R) – 2 – Amino – 1,4 – dihydro – 6 – methyl – 4 – (3 – nitrophenyl) – pyridin – 3,5 – dicarbonsäure – 3 – hexyl – 5 – [2 – (4,4 – diphenyl – 1 – piperidinyl) – ethyl] – ester,
(S) – 2 – Amino – 1,4 – dihydro – 6 – methyl – 4 – (3 – nitrophenyl) – pyridin – 3,5 – dicarbonsäure – 3 – (2 – n – butoxyethyl) – 5 – [2 – (4,4 – diphenyl – 1 – piperidinyl) – ethyl] – ester,
(R) – 2 – Amino – 1,4 – dihydro – 6 – methyl – 4 – (3 – nitrophenyl) – pyridin – 3,5 – dicarbonsäure – 3 – methyl – 5 – {2 – [4,4 – di(4 – methoxyphenyl) – 1 – piperidinyl] – ethyl} – ester,
(R) – 2 – Amino – 1,4 – dihydro – 6 – methyl – 4 – (2 – trifluormethylphenyl) – pyridin – 3,5 – dicarbonsäure – 3 – ethyl – 5 – [2 – (4,4 – diphenyl – 1 – piperidinyl) – ethyl] – ester,
(R) – 2 – Amino – 1,4 – dihydro – 6 – methyl – 4 – [3 – (1,1,2,2 – tetrafluorethoxy) – phenyl] – pyridin – 3,5 – dicarbonsäure – 3 – methyl – 5 – [2 – (4,4 – diphenyl – 1 – piperidinyl) – ethyl] – ester,
(R) – 2 – Amino – 1,4 – dihydro – 6 – methyl – 4 – (2 – difluormethoxyphenyl) – pyridin – 3,5 – dicarbonsäure – 3 – ethyl – 5 – [2 – (4,4 – diphenyl – 1 – piperidinyl) – ethyl] – ester,
(R) – 2 – Amino – 1,4 – dihydro – 6 – methyl – 4 – (2 – difluormethoxyphenyl) – pyridin – 3,5 – dicarbonsäure – 3 – ethyl – 5 – [4 – (4,4 – diphenyl – 1 – piperidinyl) – butyl] – ester,
(R) – 2 – Amino – 1,4 – dihydro – 6 – methyl – 4 – (3 – nitrophenyl) – pyridin – 3,5 – dicarbonsäure – 3 – methyl – 5 – [2 – (4,4 – dihydroxyphenyl – 1 – piperidinyl) – ethyl] – ester,
(R) – 2 – Amino – 4 – (2,3 – dichlorphenyl) – 1,4 – dihydro – 6 – methylpyridin – 3,5 – dicarbonsäure – 3 – methyl – 5 – [2 – (4,4 – diphenyl – 1 – piperidinyl) – ethyl] – ester,
(R) – 2 – Amino – 4 – (2,1,3 – benzoxdiazol – 4 – yl) – 1,4 – dihydro – 6 – methylpyridin – 3,5 – dicarbonsäure – 3 – methyl – 5 – [2 – (4,4 – diphenyl – 1 – piperidinyl) – ethyl] – ester,
(R) – 2 – Amino – 4 – (3 – cyanphenyl) – 1,4 – dihydro – 6 – methylpyridin – 3,5 – dicarbonsäure – 3 – methyl – 5 – [2 – (4,4 – diphenyl – 1 – piperidinyl) – ethyl] – ester,
(R) – 2 – Amino – 1,4 – dihydro – 6 – methyl – 4 – (2 – methoxyphenyl) – pyridin – 3,5 – dicarbonsäure – 3 – methyl – 5 – [2 – (4,4 – diphenyl – 1 – piperidinyl) – ethyl] – ester,
(R) – 2 – Amino – 1,4 – dihydro – 6 – methyl – 4 – (2 – pyridyl) – pyridin – 3,5 – dicarbonsäure – 3 – methyl – 5 – [2 – (4,4 – diphenyl – 1 – piperidinyl) – ethyl] – ester,
(R) – 2 – Amino – 1,4 – dihydro – 6 – methyl – 4 – (5 – methyl – 2 – thienyl) – pyridin – 3,5 – dicarbonsäure – 3 – methyl – 5 – [2 – (4,4 – diphenyl – 1 – piperidinyl) – ethyl] – ester,
(R) – 2 – Amino – 1,4 – dihydro – 6 – methyl – 4 – (3 – nitrophenyl) – pyridin – 3,5 – dicarbonsäure – 3 – methyl – 5 – {2 – [4 – (4 – chlorphenyl) – 4 – phenyl – 1 – piperidinyl] – ethyl} – ester,
(R) – 2 – Amino – 1,4 – dihydro – 6 – methyl – 4 – (2 – nitrophenyl) – pyridin – 3,5 – dicarbonsäure – 3 – methyl – 5 – [3 – (4,4 – diphenyl – 1 – piperidinyl) – propyl] – ester,
(R) – 2 – Amino – 1,4 – dihydro – 6 – ethyl – 4 – (3 – nitrophenyl) – pyridin – 3,5 – dicarbonsäure – 3 – methyl – 5 – [3 – (4,4 – diphenyl – 1 – piperidinyl) – propyl] – ester,
(R) – 2 – Amino – 1,4 – dihydro – 6 – methyl – 4 – (3 – nitrophenyl) – pyridin – 3,5 – dicarbonsäure – 3 – (propyl – 2) – 5 – [3 – (4,4 – diphenyl – 1 – piperidinyl) – propyl] – ester,
(R) – 2 – Amino – 1,4 – dihydro – 6 – methyl – 4 – (3 – nitrophenyl) – pyridin – 3,5 – dicarbonsäure – 3 – hexyl – 5 – [3 – (4,4 – diphenyl – 1 – piperidinyl) – propyl] – ester,
(S) – 2 – Amino – 1,4 – dihydro – 6 – methyl – 4 – (3 – nitrophenyl) – pyridin – 3,5 – dicarbonsäure – 3 – (2 – n – butoxyethyl) – 5 – [3 – (4,4 – diphenyl – 1 – piperidinyl) – propyl] – ester,
(R) – 2 – Amino – 1,4 – dihydro – 6 – methyl – 4 – (3 – nitrophenyl) – pyridin – 3,5 – dicarbonsäure – 3 – methyl – 5 – {3 – [4,4 – di(4 – methoxyphenyl) – 1 – piperidinyl] – propyl} – ester,
(R) – 2 – Amino – 1,4 – dihydro – 6 – methyl – 4 – (2 – trifluormethylphenyl) – pyridin – 3,5 – dicarbonsäure – 3 – ethyl – 5 – [3 – (4,4 – diphenyl – 1 – piperidinyl) – propyl] – ester,
(R) – 2 – Amino – 1,4 – dihydro – 6 – methyl – 4 – [3 – (1,1,2,2 – tetrafluorethoxy) – phenyl] – pyridin – 3,5 – dicarbonsäure – 3 – methyl – 5 – [3 – (4,4 – diphenyl – 1 – piperidinyl) – propyl] – ester,
(R) – 2 – Amino – 1,4 – dihydro – 6 – methyl – 4 – (2 – difluormethoxyphenyl) – pyridin – 3,5 – dicarbonsäure – 3 – ethyl – 5 – [3 – (4,4 – diphenyl – 1 – piperidinyl) – propyl] – ester,
(R) – 2 – Amino – 1,4 – dihydro – 6 – methyl – 4 – (3 – nitrophenyl) – pyridin – 3,5 – dicarbonsäure – 3 – methyl – 5 – [3 – (4,4 – dihydroxyphenyl – 1 – piperidinyl) – propyl] – ester,
(R) – 2 – Amino – 4 – (2,3 – dichlorphenyl) – 1,4 – dihydro – 6 – methylpyridin – 3,5 – dicarbonsäure – 3 – methyl – 5 – [3 – (4,4 – diphenyl – 1 – piperidinyl) – propyl] – ester,
(R) – 2 – Amino – 4 – (2,1,3 – benzoxdiazol – 4 – yl) – 1,4 – dihydro – 6 – methylpyridin – 3,5 – dicarbonsäure – 3 – methyl – 5 – [3 – (4,4 – diphenyl – 1 – piperidinyl) – propyl] – ester,
(R) – 2 – Amino – 4 – (3 – cyanphenyl) – 1,4 – dihydro – 6 – methylpyridin – 3,5 – dicarbonsäure – 3 – methyl –

5 – [3 – (4,4 – diphenyl – 1 – piperidinyl) – propyl] – ester,
(R) – 2 – Amino – 1,4 – dihydro – 6 – methyl – 4 – (2 – methoxyphenyl) – pyridin – 3,5 – dicarbonsäure – 3 – methyl – 5 – [3 – (4,4 – diphenyl – 1 – piperidinyl) – propyl] – ester,
(R) – 2 – Amino – 1,4 – dihydro – 6 – methyl – 4 – (2 – pyridyl) – pyridin – 3,5 – dicarbonsäure – 3 – methyl – 5 – [3 – (4,4 – diphenyl – 1 – piperidinyl) – propyl] – ester,
(R) – 1,4 – Dihydro – 2,6 – dimethyl – 4 – (3 – nitrophenyl) – pyridin – 3,5 – dicarbonsäure – 3 – ethyl – 5 – {2 – [2 – (4,4 – diphenyl – 1 – piperidinyl) – ethoxi] – ethyl} – ester,
(S) – 1,4 – Dihydro – 2,6 – dimethyl – 4 – (3 – nitrophenyl) – pyridin – 3,5 – dicarbonsäure – 3 – (2 – methoxyethyl) – 5 – {3 – [3 – (4,4 – diphenyl – 1 – piperidinyl) – propoxi] – propyl} – ester,
(R) – 1,4 – Dihydro – 2,6 – dimethyl – 4 – (2 – nitrophenyl) – pyridin – 3,5 – dicarbonsäure – 3 – methyl – 5 – {2 – [2 – (4,4 – diphenyl – 1 – piperidinyl) – ethoxi] – ethyl} – ester,
(R) – 1,4 – Dihydro – 2,6 – diethyl – 4 – (3 – nitrophenyl) – pyridin – 3,5 – dicarbonsäure – 3 – methyl – 5 – {2 – [2 – (4,4 – diphenyl – 1 – piperidinyl) – ethoxi] – ethyl} – ester,
(R) – 1,4 – Dihydro – 2,6 – dimethyl – 4 – (3 – nitrophenyl) – pyridin – 3,5 – dicarbonsäure – 3 – (propyl – 2) – 5 – {2 – [2 – (4,4 – diphenyl – 1 – piperidinyl) – ethoxi] – ethyl} – ester,
(R) – 1,4 – Dihydro – 2,6 – dimethyl – 4 – (3 – nitrophenyl) – pyridin – 3,5 – dicarbonsäure – 3 – hexyl – 5 – {2 – [2 – (4,4 – diphenyl – 1 – piperidinyl) – ethoxi] – ethyl} – ester,
(R) – 1,4 – Dihydro – 2,6 – dimethyl – 4 – (3 – nitrophenyl) – pyridin – 3,5 – dicarbonsäure – 3 – (2 – n – butoxyethyl) – 5 – {2 – [2 – (4,4 – diphenyl – 1 – piperidinyl) – ethoxi] – ethyl} – ester,
(R) – 1,4 – Dihydro – 2,6 – dimethyl – 4 – (2 – trifluormethylphenyl) – pyridin – 3,5 – dicarbonsäure – 3 – ethyl – 5 – {2 – [2 – (4,4 – diphenyl – 1 – piperidinyl) – ethoxi] – ethyl} – ester,
(R) – 1,4 – Dihydro – 2,6 – dimethyl – 4 – [3 – (1,1,2,2 – tetrafluorethoxy) – phenyl] – pyridin – 3,5 – dicarbonsäure – 3 – methyl – 5 – {2 – [2 – (4,4 – diphenyl – 1 – piperidinyl) – ethoxi] – ethyl} – ester,
(R) – 1,4 – Dihydro – 2,6 – dimethyl – 4 – (2 – difluormethoxyphenyl) – pyridin – 3,5 – dicarbonsäure – 3 – ethyl – 5 – {2 – [2 – (4,4 – diphenyl – 1 – piperidinyl) – ethoxi] – ethyl} – ester,
(R) – 1,4 – Dihydro – 2,6 – dimethyl – 4 – (3 – nitrophenyl) – pyridin – 3,5 – dicarbonsäure – 3 – methyl – 5 – {2 – [2 – (4,4 – diphenyl – 1 – piperidinyl) – ethoxi] – ethyl} – ester,
(R) – 4 – (2,3 – Dichlorphenyl) – 1,4 – dihydro – 2,6 – dimethylpyridin – 3,5 – dicarbonsäure – 3 – methyl – 5 – {2 – [2 – (4,4 – diphenyl – 1 – piperidinyl) – ethoxi] – ethyl} – ester,
(R) – 4 – (2,1,3 – Benzoxdiazol – 4 – yl) – 1,4 – dihydro – 2,6 – dimethylpyridin – 3,5 – dicarbonsäure – 3 – methyl – 5 – {2 – [2 – (4,4 – diphenyl – 1 – piperidinyl) – ethoxi] – ethyl} – ester,
(R) – 4 – (3 – Cyanphenyl) – 1,4 – dihydro – 2,6 – dimethylpyridin – 3,5 – dicarbonsäure – 3 – methyl – 5 – {2 – [2 – (4,4 – diphenyl – 1 – piperidinyl) – ethoxi] – ethyl} – ester,
(R) – 1,4 – Dihydro – 2,6 – dimethyl – 4 – (2 – methoxyphenyl) – pyridin – 3,5 – dicarbonsäure – 3 – methyl – 5 – {2 – [2 – (4,4 – diphenyl – 1 – piperidinyl) – ethoxi] – ethyl} – ester,
(R) – 1,4 – Dihydro – 2,6 – dimethyl – 4 – (2 – pyridyl) – pyridin – 3,5 – dicarbonsäure – 3 – methyl – 5 – {2 – [2 – (4,4 – diphenyl – 1 – piperidinyl) – ethoxi] – ethyl} – ester,
(R) – 1,4 – Dihydro – 2,6 – dimethyl – 4 – (5 – methyl – 2 – thienyl) – pyridin – 3,5 – dicarbonsäure – 3 – methyl – 5 – {2 – [2 – (4,4 – diphenyl – 1 – piperidinyl) – ethoxi] – ethyl} – ester,
(R) – 1,4 – Dihydro – 2,6 – dimethyl – 4 – (2 – nitrophenyl) – pyridin – 3,5 – dicarbonsäure – 3 – methyl – 5 – {3 – [3 – (4,4 – diphenyl – 1 – piperidinyl) – propoxi] – propyl} – ester,
(R) – 1,4 – Dihydro – 2,6 – diethyl – 4 – (3 – nitrophenyl) – pyridin – 3,5 – dicarbonsäure – 3 – methyl – 5 – {3 – [3 – (4,4 – diphenyl – 1 – piperidinyl) – propoxi] – propyl} – ester,
(R) – 1,4 – Dihydro – 2,6 – dimethyl – 4 – (3 – nitrophenyl) – pyridin – 3,5 – dicarbonsäure – 3 – (propyl – 2) – 5 – {3 – [3 – (4,4 – diphenyl – 1 – piperidinyl) – propoxi] – propyl} – ester,
(R) – 1,4 – Dihydro – 2,6 – dimethyl – 4 – (3 – nitrophenyl) – pyridin – 3,5 – dicarbonsäure – 3 – hexyl – 5 – {3 – [3 – (4,4 – diphenyl – 1 – piperidinyl) – propoxi] – propyl} – ester,
(S) – 1,4 – Dihydro – 2,6 – dimethyl – 4 – (3 – nitrophenyl) – pyridin – 3,5 – dicarbonsäure – 3 – (2 – n – butoxyethyl) – 5 – {3 – [3 – (4,4 – diphenyl – 1 – piperidinyl) – propoxy] – propyl} – ester,
(R) – 1,4 – Dihydro – 2,6 – dimethyl – 4 – (2 – trifluormethylphenyl) – pyridin – 3,5 – dicarbonsäure – 3 – ethyl – 5 – {3 – [3 – (4,4 – diphenyl – 1 – piperidinyl) – propoxi] – propyl} – ester,
(R) – 1,4 – Dihydro – 2,6 – dimethyl – 4 – [3 – (1,1,2,2 – tetrafluorethoxy) – phenyl] – pyridin – 3,5 – dicarbonsäure – 3 – methyl – 5 – {3 – [3 – (4,4 – diphenyl – 1 – piperidinyl) – propoxi] – propyl} – ester,
(R) – 1,4 – Dihydro – 2,6 – dimethyl – 4 – (2 – difluormethoxyphenyl) – pyridin – 3,5 – dicarbonsäure – 3 – ethyl – 5 – {3 – [3 – (4,4 – diphenyl – 1 – piperidinyl) – propoxi] – propyl} – ester,
(R) – 1,4 – Dihydro – 2,6 – dimethyl – 4 – (3 – nitrophenyl) – pyridin – 3,5 – dicarbonsäure – 3 – methyl – 5 – {3 – [3 – (4,4 – dihydroxyphenyl – 1 – piperidinyl) – propoxi] – propyl} – ester,
(R) – 4 – (2,3 – Dichlorphenyl) – 1,4 – dihydro – 2,6 – dimethylpyridin – 3,5 – dicarbonsäure – 3 – methyl – 5 –

{3 – [3 – (4,4 – diphenyl – 1 – piperidinyl) – propoxi] – propyl} – ester,
(R) – 4 – (2,1,3 – Benzoxdiazol – 4 – yl) – 1,4 – dihydro – 2,6 – dimethylpyridin – 3,5 – dicarbonsäure – 3 – methyl – 5 – {3 – [3 – (4,4 – diphenyl – 1 – piperidinyl) – propoxi] – propyl} – ester,
(R) – 4 – (3 – Cyanphenyl) – 1,4 – dihydro – 2,6 – dimethylpyridin – 3,5 – dicarbonsäure – 3 – methyl – 5 – {3 – [3 – (4,4 – diphenyl – 1 – piperidinyl) – propoxi] – propyl} – ester,
(R) – 1,4 – Dihydro – 2,6 – dimethyl – 4 – (2 – methoxyphenyl) – pyridin – 3,5 – dicarbonsäure – 3 – methyl – 5 – {3 – [3 – (4,4 – diphenyl – 1 – piperidinyl) – propoxi] – propyl} – ester,
(R) – 1,4 – Dihydro – 2,6 – dimethyl – 4 – (2 – pyridyl) – pyridin – 3,5 – dicarbonsäure – 3 – methyl – 5 – {3 – [3 – (4,4 – diphenyl – 1 – piperidinyl) – propoxi] – propyl} – ester und
(R) – 1,4 – Dihydro – 2,6 – dimethyl – 4 – (5 – methyl – 2 – thienyl) – pyridin – 3,5 – dicarbonsäure – 3 – methyl – 5 – {3 – [3 – (4,4 – diphenyl – 1 – piperidinyl) – propoxi] – propyl} – ester,
und ihrer Salze.

Besonders bevorzugt ist die erfindungsgemäße Verwendung der Verbindung (R) – ( + ) – 1,4 – Dihydro – 2,6 – dimethyl – 4 – (3 – nitrophenyl) – pyridin – 3,5 – dicarbonsäure – 3 – methyl – 5 – [3 – (4,4 – diphenyl – 1 – piperidinyl) – propyl] – ester, und ihrer Salze.

Besonders bevorzugter Gegenstand der Erfindung ist die Verwendung solcher optisch reiner 1,4 – Dihydropyridine der Formel I, die – insbesondere im Vergleich zu ihren optischen Antipoden – nur einen schwachen Einfluß auf das cardiovaskuläre System haben.

Die Herstellung der Verbindungen der Formel I ist beispielsweise in der Europäischen Patentanmeldung 0 242 829 beschrieben. Die Verbindungen I können auch hergestellt werden, indem man optisch reine Dihydropyridinderivate der Formel II

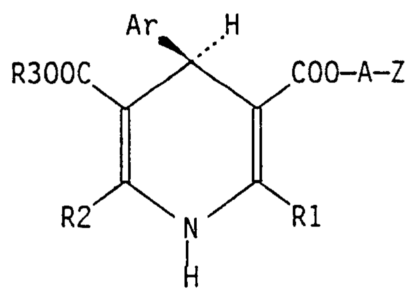

(II)

mit Diarylpiperidinen der Formel III

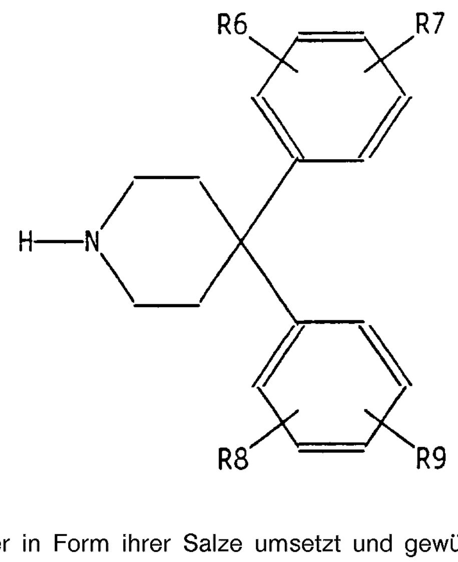

(III)

als solche(n) oder in Form ihrer Salze umsetzt und gewünschtenfalls anschließend erhaltene Salze in die freien Basen oder erhaltene Basen in die Salze überführt, wobei Ar, R1, R2, R3, R6, R7, R8, R9 und A die oben angegebenen Bedeutungen haben und Z eine geeignete Abgangsgruppe darstellt.

Die Umsetzung wird in geeigneten, vorzugsweise inerten organischen Lösungsmitteln in Gegenwart von Wasser oder ohne Wasser durchgeführt. Beispielsweise seien genannt Ether, wie Dioxan, Diethylether, Tetrahydrofuran, Glykolmonoethylether oder Glykoldimethylether; Ketone, wie Aceton oder Ethylmethylke –

ton; aromatische Kohlenwasserstoffe, wie Xylol oder Toluol; oder chlorierte Kohlenwasserstoffe, wie Methylenchlorid, Chloroform, Tetrachlorethylen oder Dichlorethan; oder polare aprotische Lösungsmittel wie Dimethylformamid, N-Methylpyrrolidon oder Dimethylsulfoxid.

Die Reaktionstemperaturen können - je nach Reaktivität der Edukte - in einem weiten Bereich variieren. Im allgemeinen wird die Umsetzung bei Temperaturen zwischen 20°C und 150°C, vorzugsweise zwischen 20°C und 100°C, insbesondere bei der Siedetemperatur des verwendeten Lösungsmittels durchgeführt.

Das Verfahren kann bei Normaldruck oder bei erhöhtem Druck durchgeführt werden, wobei das Arbeiten bei Normaldruck die Regel ist.

Je nach Art der Abgangsgruppe Z, die beispielsweise ein Tosyl- oder Triflatrest, vorzugsweise ein Halogenatom, insbesondere ein Bromatom ist, kann die Reaktion gewünschtenfalls in Gegenwart einer Base (z.B. eines anorganischen Carbonates, wie Kaliumcarbonat) oder unter Einsetzung eines Überschusses an Diarylpiperidin III durchgeführt werden.

Die Isolierung und Reinigung der Verbindungen I erfolgt in an sich bekannter Weise z. B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), oder in einem offenkettigen oder cyclischen Ether, wie Dioxan oder Tetrahydrofuran, das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen II werden ausgehend von optisch reinen Dihydropyridincarbonsäuren IV

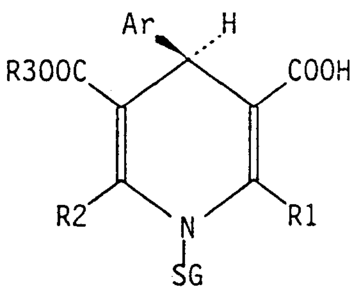

(IV)

durch Umsetzung mit geeigneten bifunktionellen Alkylderivaten V

Z-A-Z (V)

und anschließende Abspaltung von SG erhalten, wobei Ar, R1, R2, R3 und A die oben angegebenen Bedeutungen haben, Z eine geeignete Abgangsgruppe und SG eine Schutzgruppe darstellt.

Die Umsetzung von IV mit V erfolgt bevorzugt unter basischen Bedingungen in Gegenwart eines Phasentransferkatalysators.

Als Katalysatoren seien neben Oniumsalzen, wie z.B. Tetrabutylammoniumbromid oder Benzyltriethylammoniumchlorid, vor allem Kronenether, wie Dibenzo-[18]krone-6, Dicyclohexyl-[18]krone-6 und insbesondere [18]Krone-6 erwähnt.

Als eingesetzte Basen, die wenigstens im molaren Verhältnis, vorzugsweise im Überschuß eingesetzt werden, kommen anorganische Basen, wie Alkalimetallhydroxide (z.B. Natrium- oder Kaliumhydroxid), oder insbesondere Alkalimetallcarbonate (z.B. Natrium- oder vorzugsweise Kaliumcarbonat) in Frage. Beim Arbeiten in einem wasserfreien Lösungsmittel werden die verwendeten Hydroxide bzw. Carbonate vorzugsweise in feingepulverter Form eingesetzt.

Die Umsetzung erfolgt (je nach Art des Phasentransferkatalysators, der Abgangsgruppe Z und der eingesetzten Base) in wasserhaltigen oder wasserfreien organischen Lösungsmitteln, oder in einem Gemisch aus Wasser und einem mit Wasser nicht oder kaum mischbaren organischen Lösungsmittel. Als Wasser/Lösungsmittelmischungen seien beispielsweise die Mischungen von Wasser mit Chloroform, Dichlormethan oder Benzol genannt. Als wasserhaltige oder wasserfreie Lösungsmittel seien beispielsweise Dichlormethan, Acetonitril oder Aceton genannt. Die Abgangsgruppe Z ist vorzugsweise ein Halogenatom, insbesondere ein Bromatom.

Die Wahl der Reaktionstemperatur bei der Umsetzung von IV mit V hängt von den übrigen Reaktionsbedingungen ab, wobei in der Regel Temperaturen zwischen 20°C und der Siedetemperatur des eingesetzten Lösungsmittels bevorzugt sind.

Als Schutzgruppen SG kommen vor allem solche Gruppen in Frage, die in das der Verbindung IV zugrundeliegende Vorprodukt leicht und in hohen Ausbeuten eingeführt werden können, die bei der Umsetzung von IV mit V keine Nebenreaktionen eingehen und die am Ende glatt wieder abgespalten werden können. Als bevorzugte Schutzgruppen SG seien beispielsweise Alkoxymethylgruppen oder Benzyloxymethylgruppen, insbesondere die Ethoxymethylgruppe genannt. Die Abspaltung der Schutzgruppe erfolgt in saurem Medium, beispielsweise in 1N Salzsäure oder vorzugsweise in wasserfreier Ameisensäure, unter Reaktionsbedingungen, wie sie dem Fachmann geläufig sind. Die Abspaltung der Schutzgruppe kann auch nach der Umsetzung mit dem Diarylpiperidin III erfolgen.

Die in den Beispielen genannten Lösungsmittel, Basen und Phasentransferkatalysatorenstellen nur eine exemplarische Auswahl dar. Welche weiteren Kombinationen von Lösungsmitteln, Basen und Phasentransferkatalysatoren noch geeignet sind, ist dem Fachmann aufgrund seines Fachwissens geläufig.

Die Dihydropyridincarbonsäuren IV sind aus Chem.Pharm.Bull. 28(9), 2809 – 2812 (1980) bekannt bzw. sie können in analoger Weise wie dort beschrieben hergestellt werden. Die. Diarylpiperidine III sind z. B. aus der DE – OS 19 36 452 bekannt. Die bifunktionellen Alkylderivate V sind bekannt oder sie können nach bekannten Verfahren hergestellt werden.

Die folgenden Herstellungsbeispiele sollen die Erfindung näher erläutern, ohne sie einzuschränken. Schmp. bedeutet Schmelzpunkt, h steht für Stunden, Kp. steht für Siedepunkt, Zers. bedeutet Zersetzung.

## Beispiele

### Endprodukte

1. (–)-1,4-Dihydro-2,6-dimethyl-4-(3-nitrophenyl)-pyridin-3,5-dicarbonsäure-3-methyl-5-[3-(4,4-diphenyl-1-piperidinyl)-propyl]ester-hydrochlorid

86,6 g (–)-1,4-Dihydro-2,6-dimethyl-4-(3-nitrophenyl)-pyridin-3,5-dicarbonsäure-3-methyl-5-(3-brompropyl)-ester, 50 g 4,4-Diphenylpiperidin-hydrochlorid und 69 g feingemahlenes Kaliumcarbonat werden zusammen in 300 ml Dimethylformamid unter kräftigem Rühren und unter einer Stickstoffathmosphäre 5 h auf 100°C erhitzt. Nach Abkühlen werden nacheinander 500 ml Ethylacetat und 1 l Wasser zugegeben und kräftig ausgerührt. Nach Phasentrennung wird die organische Phase noch viermal mit Wasser gewaschen, über Natriumsulfat getrocknet und im Vakuum eingeengt. Der Rückstand wird in 1 l Dioxan gelöst und die Lösung unter Kühlen mit 15,2 ml konzentrierter Salzsäurelösung (12,5 M, d = 1,19) versetzt; dann werden im Vakuum ca. 200 ml des Lösungsmittelgemisches abdestilliert. Beim Stehen bei Raumtemperatur kristallisiert das Produkt spontan oder nach Animpfen oder Anreiben; es wird nach 16 h abgesaugt, mit Dioxan und Diisopropylether gewaschen und im Vakuum bei 80 – 100°C getrocknet. Zur Reinigung wird das Rohprodukt in Dichlormethan gelöst. Nach Zugabe von 800 ml Dioxan wird das Dichlormethan wieder abdestilliert. Das nach Animpfen und 16-stündigem Stehen bei Raumtemperatur auskristallisierte Produkt wird abgesaugt, mit Dioxan und Diisopropylether gewaschen und bei 100°C getrocknet. Man erhält 97 g der Titelverbindung vom Schmp. 158 – 160°C mit

$$[\alpha]^{22}_{436} = -39^{o}$$

(c = 1, Methanol) bzw. $[\alpha]^{22}_{D}$ = – 14,4° (c = 1, Methanol).

Alternativ wird die Titelverbindung wie folgt hergestellt:

64,6 g (±) – 1,4 – Dihydro – 2,6 – dimethyl – 4 – (3 – nitrophenyl) – pyridin – 3,5 – dicarbonsäure – 3 – methyl – 5 – [3 – (4,4 – diphenyl – 1 – piperidinyl) – propyl] – ester – hydrochlorid werden in 300 ml Dichlor – methan gelöst. Die Lösung wird mit 100 ml konzentrierter Ammoniak – Lösung und zweimal mit je 100 ml Wasser gewaschen. Nach dem Trocknen der organischen Phase über Natriumsulfat engt man zur Trockene ein. Der erhaltene Rückstand (60,91 g) und 37,63 g L – ( – ) – 0,0' – Dibenzoylweinsäurehydrat (mit $[\alpha]_D^{22}$ = – 108,1°; c = 1, Methanol) werden in 400 ml Ethanol in der Siedehitze gelöst. Beim langsamen Abkühlen der gerührten Lösung erhält man als erstes Kristallisat 55 g 0,0' – Dibenzoyltartrat der Titelverbindung als feine gelbliche Kristalle, die erneut in der Siedehitze in einem Gemisch aus Chloroform/Methanol (4 + 1) gelöst werden. Nachdem sich die Kristalle gerade gelöst haben, versetzt man die siedende Lösung mit Essigsäureethylester (20 Vol. – % des Chlorform/Methanol – Gemisches) und läßt unter Rühren langsam abkühlen. Das erhaltene feine zweite Kristallisat (50 g) wird auf die gleiche Weise noch dreimal umkristalli – siert. Man erhält nacheinander ein drittes (36 g mit $[\alpha]_D^{22}$ = – 47,5°), viertes (33 g mit $[\alpha]_D^{22}$ = – 49,4°) und fünftes {31 g mit $[\alpha]_D^{22}$ = – 50,4° (c = 1, Methanol)} Kristallisat. Das fünfte Kristallisat wird in 500 ml Dichlormethan gelöst und die Lösung zweimal mit je 150 ml konzentrierter Ammoniak – Lösung und dreimal mit je 100 ml Wasser gewaschen. Nach dem Einengen der organischen Phase wird der verbleibende Rückstand wie oben beschrieben aufgearbeitet. Ausbeute: 19,8 g, $[\alpha]_D^{22}$ = – 14,3°, (c = 1, Methanol).

2. <u>( + ) – 2 – Amino – 1,4 dihydro – 6 – methyl – 4 – (3 – nitrophenyl) – pyridin – 3,5 – dicarbonsäure – 3 – ethyl – 5 – [3 – (4,4 – diphenyl – 1 – piperidinyl) – propyl] – ester – fumarat</u>

60 g (±) – 2 – Amino – 1,4 – dihydro – 6 – methyl – 4 – (3 – nitrophenyl) – pyridin – 3,5 – dicarbonsäure – 3 – ethyl – 5 – [3 – (4,4 – diphenyl – 1 – piperidinyl) – propyl] – ester und 36,2 g L – ( – ) – 0,0' – Dibenzoylweinsäure – hydrat {in diesem Beispiel mit der spezifischen Drehung $[\alpha]_D^{22}$ = – 108,4° (c = 1, Methanol)} werden zusammen in Methanol/Dichlormethan (9 + 1) gelöst. Nach dem Einengen der Lösung nimmt man den fest aufgeschäumten Rückstand in der Siedehitze mit 300 ml Methylethylketon/Methanol (2 + 1) auf und läßt die klare Lösung unter gutem Rühren langsam abkühlen. Man erhält ein erstes Kristallisat {33 g, Schmp. 165 – 166°C, $[\alpha]_D^{22}$ = – 17,8° (c = 1, Methanol)}, das erneut in Methylethylketon/(Methanol (2 + 1) umkristallisiert ein zweites Kristallisat ergibt {22 g, $[\alpha]_D^{22}$ = – 10,8° (c = 1, Methanol)}. Die groben, gelblichen Nadeln löst man in 400 ml Dichlormethan und extrahiert die Lösung mit 300 ml konzentrierter Ammoniaklösung und anschließend dreimal mit je 100 ml Wasser. Nach dem Trocknen der organischen Phase über Natriumsulfat engt man ein. Der erhaltene fest aufgeschäumte Rückstand (14,3 g) wird zusammen mit 2,6 g Fumarsäure in Methanol gelöst und die Lösung erneut eingeengt. Der Rückstand wird in siedendem Essigsäureethylester/2 – Propanol (9 + 1) gelöst. Nach dem langsamen Abkühlen der Lösung erhält man 12,4 g der Titelverbindung als feine Nadeln vom Schmp. 151 – 152°C, $[\alpha]_D^{22}$ = + 42,0° (c = 1,

Die nach der Extraktion mit Ammoniak erhaltene freie Base der Titelverbindung kann in Petrolether amorph ausgefällt werden. Man erhält ein feines gelbliches Pulver vom Schm. 96 – 104°C (langsames Zerfließen) , $[\alpha]_D^{22}$ = + 57,6° (c = 1, Methanol).

3. <u>( + ) – 1,4 – Dihydro – 2,6 – dimethyl – 4 – (3 – nitrophenyl) – pyridin – 3,5 – dicarbonsäure – 3 – methyl – 5 – {2 – [2 – (4,4 – diphenyl – 1 – piperidinyl) – ethoxi] – ethyl} – ester – hydrochlorid</u>

997 mg ( + ) – 1,4 – Dihydro – 2,6 – dimethyl – 4 – (3 – nitrophenyl) – pyridin – 3,5 – dicarbonsäure – 3 – methylester werden mit 3 ml Oxalylchlorid versetzt. Die Mischung wird bei Raumtemperatur solange gerührt, bis keine weitere Gasentwicklung mehr erkennbar ist. Unter Zusatz von je 5 ml abs. Toluol engt man den Ansatz dreimal zur Trockene ein. Der erhaltene braune feste Rückstand wird in 3 ml abs. Dichlormethan suspendiert und die Suspension unter $N_2$ – Begasung in eine auf 0°C gekühlte Lösung von 1,09 g N – [2 – (2 – Hydroxiethoxi) – ethyl] – 4,4 – diphenylpiperidin und 0,6 ml Triethylamin eingetropft. Nach dem Zutropfen rührt man die Mischung noch 2 h bei Raumtemperatur und engt dann zur Trockene ein. Der verbleibende bräunliche Rückstand wird in 100 ml Dichlormethan aufgenommen und dreimal mit je 50 ml Wasser extrahiert. Nach dem Trocknen der organischen Phase über Natriumsulfat engt man die bräunliche klare Lösung weitgehend ein und chromatographiert den öligen Rückstand über eine 2 x 30 cm Kieselgel – säule mit Dichlormethan/Ethanol (98 + 2) als Elutionsmittel. Nach dem Einengen der chromatographisch einheitlichen Produktfraktion nimmt man den verbleibenden gelblichen Rückstand in 5 ml Dichlormethan auf und versetzt die Lösung mit etherischer Salzsäure Nach erneutem Einengen der Hydrochloridlösung zur Trockene wird der fest aufgeschäumte Rückstand in 3 ml Dichlormethan gelöst und das Produkt durch Eintropfen der Lösung in 1 l Petrolether/Diethylether (2 + 1) amorph ausgefällt. Nach Absaugen und

Trocknen des Niederschlages erhält man die Titelverbindung als feines graues Pulver vom Schmp.: 118–128°C (langsames Zerfließen); $[\alpha]_D^{22}$ = + 0,9° (c = 1, Methanol); Ausbeute: 490 mg.

Ausgangsverbindung

()–1,4–Dihydro–2,6–dimethyl–4–(3–nitrophenyl)–pyridin–3,5–dicarbonsäure–3–methyl–5–(3–brompropyl)–ester

168,5 g (+)–1–Ethoxymethyl–1,4–dihydro–5–methoxycarbonyl–2,6–dimethyl–4–(3–nitrophenyl)–pyridin–3–carbonsäure–Cinchoninsalz {$[\alpha]_D^{22}$ = + 101,50° (c = 1, Chloroform)} wird in 1,5 l Dichlormethan gelöst und dann unter Kühlung und kräftigem Rühren mit 1,2 l 0,2 N Salzsäurelösung versetzt. Durch Zugabe von 2 N Salzsäurelösung wird in der wässerigen Phase ein stabiler pH von 2 eingestellt. Nach Phasentrennung wird die organische Phase noch viermal mit Salzsäurelösung von pH 2 und dann mit Wasser gewaschen, über Natriumsulfat getrocknet und eingeengt. Der ölige Rückstand wird in 1,1 l Aceton gelöst und mit 375 g feinvermahlenem Kaliumcarbonat, 375 ml 1,3–Dibrompropan und 1,2 g [18] Krone–6 versetzt. Das Gemisch wird 24 h kräftig bei Raumtemperatur gerührt; dann wird abgesaugt und der Filterkuchen mit Aceton gewaschen. Das Filtrat wird am Rotationsverdampfer bei schwachem Vakuum eingeengt und dann das überschüssige 1,3–Dibrompropan bei 0,02 mbar abdestilliert (Badtemperatur bis 45°C). Der ölige Rückstand wird unter Eiskühlung mit 690 ml konzentrierter Ameisensäure übergossen, dann wird bei Raumtemperatur solange gerührt, bis eine klare Lösung entstanden ist (ca. 15 Min). Die Ameisensäure wird im Vakuum abdestilliert. Nach zweimaliger Zugabe und Abdestillieren von je 200 ml Toluol wird der Rückstand in 900 ml Dichlormethan gelöst. Die Lösung wird mit Natriumhydrogencarbonatlösung (pH 8,5) ausgerührt, mit Wasser gewaschen, über Natriumsulfat getrocknet und im Vakuum eingeengt. Das nach Zugabe von Diisopropylether spontan kristallisierende Produkt wird abgesaugt, mit Diisopropvlether gewaschen und im Vakuum getrocknet. Man erhält 102 g der Titelverbindung vom Schmp. 112–114°C und $[\alpha]_D^{22}$ = – 13,8° (c = 1, Methanol).

Gewerbliche Anwendbarkeit

Die Verbindungen der Formel I und ihre Salze besitzen wertvolle Eigenschaften, die sie gewerblich verwertbar machen. Sie stellen in erster Linie antineoplastisch wirksame Agenzien mit interessanter cytostatischer Aktivität dar. Sie können bei der Behandlung von Tumorerkrankungen eingesetzt werden, beispielsweise zur Verringerung oder Verhinderung der Metastasenbildung und des Tumorwachstums bei Säugern.

In ihrer ausgezeichneten Wirksamkeit, die in einer selektiven, kontrollierten Proliferationshemmung zum Ausdruck kommt und die gepaart ist mit geringer Toxizität und dem Fehlen unerwünschter Nebenwirkungen, unterscheiden sich Verbindungen der Formel I und ihre Salze in überraschender und vorteilhafter Weise von solchen 1,4–Dihydropyridinen, deren Verwendung als Krebschemotherapeutika in der Literatur vorgeschlagen wird. Es muß besonders darauf hingewiesen werden, daß bisher nur 1,4–Dihydropyridine mit ausgeprägten calciumkanal–blockierenden Eigenschaften als für die Krebschemotherapie geeignet betrachtet wurden, das heißt, die calciumkanal–blockierende (calciumantagonistische) Aktivität galt als Grundvoraussetzung für die cytostatische Aktivität. Es wurde nun überraschenderweise gefunden, daß Verbindungen der Formel I und ihre Salze, die nur eine geringe calciumkanal–blockierende Wirkung aufweisen, die ausgeprägte Fähigkeit besitzen, das Wachstum von Tumorzellen in vitro zu hemmen, woraus auf eine entsprechende in vivo–Wirkung geschlossen werden kann.

Die geringe calciumkanal–blockierende Aktivität von Verbindungen der Formel I kommt in dem vergleichsweise geringen Einfluß dieser Verbindungen auf das cardiovaskuläre System, z.B. auf den Blutdruck und die Herzfrequenz, zum Ausdruck. Diese schwache cardiovaskuläre Aktivität von Verbindungen der Formel I und ihren Salzen gestattet ihren Einsatz in der Humanmedizin als potente Mittel zur Tumorwachstumshemmung und Verhinderung der Metastasenbildung. Im Gegensatz zu den cardiovaskulär ausgeprägt wirksamen Calciumkanalblockern, die bisher als antineoplastisch wirksame Agenzien bekannt waren, können Verbindungen der Formel I und ihre Salze in therapeutisch wirksamen Dosen ohne Gefahr unerwünschter Nebenwirkungen auf das cardiovaskuläre System verabfolgt werden.

Die ausgezeichnete Wirksamkeit von Verbindungen der Formel I und ihren Salzen gestattet ihren Einsatz in der Humanmedizin als Chemotherapeutika für die Behandlung von Tumoren, z.B. von Ovarialkarzinomen, Hodentumoren, Prostatakarzinomen, Blasentumoren, Ösophaguskarzinomen und anderen bösartigen Gewebsneubildungen, insbesondere von Darmkrebs, Brustkrebs, Bronchialcarcinomen und Lungencarcinomen.

Die Erfindung umfaßt die Verwendung von Verbindungen der Formel I und ihren pharmakologisch verträglichen Salze bei der Herstellung von Arzneimitteln, die zur Bekämpfung der genannten Krankheiten eingesetzt werden.

Die Arzneimittel werden nach an sich bekannten, dem Fachmann geläufigen Verfahren hergestellt. Als Arzneimittel werden die pharmakologisch wirksamen Verbindungen der Formel I und ihre Salze (=Wirkstoffe) entweder als solche, oder vorzugsweise in Kombination mit geeigneten pharmazeutischen Hilfsstoffen in Form von Tabletten, Dragees, Kapseln, Suppositorien, Pflastern (zur transdermalen Arzneiapplikation), Emulsionen, Suspensionen, Aerosolen, Sprays, Salben, Cremes, Gelen oder Lösungen eingesetzt, wobei der Wirkstoffgehalt vorteilhafterweise zwischen 0,1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können rektal, per inhalationem, parenteral (perlingual, intravenös, percutan) oder oral appliziert werden.

Im allgemeinen hat es sich in der Humanmedizin als vorteilhaft erwiesen, den oder die Wirkstoffe bei oraler Gabe in einer Tagesdosis von etwa 0,5 bis 30 mg/kg Körpergewicht, gewünschtenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre pharmakologisch verträglichen Salze zur Behandlung der genannten Krankheiten eingesetzt werden, so können die pharmazeutischen Zubereitungen auch einen oder mehrere andere pharmakologisch aktive Bestandteile anderer Arzneimittelgruppen enthalten.

Wie bei der internistischen Tumortherapie üblich, kann zur Reduzierung des Nebenwirkungsrisikos die Behandlung mit den erfindungsgemäßen Arzneimittel kombiniert werden mit der Verabreichung anderer Cytostatica mit unterschiedlichen Wirkungsspektren. Es kann auch zweckmäßig sein, die Behandlung nach dem Prinzip der zyklischen Cytostaticatherapie durchzuführen. Hierbei wird nach jeder Behandlung eine Erholungsphase eingelegt. Man macht sich dabei die Erfahrung zunutze, daß gesundes Gewebe der meisten Organe schneller regeneriert als malignes Gewebe.

Pharmakologie

Die tumorwachstumshemmende Aktivität von (+)-1,4-Dihydro-2,6-dimethyl-4-(3-nitrophenyl)-pyridin-3,5-dicarbonsäure-3-methyl-5-[3-(4,4-diphenyl-1-piperidinyl)-propyl]ester (Verbindung 1) wurde in verschiedenen in vitro Testsystemen untersucht. Die Tests und die Testresultate werden näher wie folgt beschrieben:

1. Dosis-Wirkungsbeziehung des tumorwachstumshemmenden Effektes von 1 in der von menschlichen Lungentumoren abgeleiteten Zellinie NCI-H 727

Methoden und Ergebnisse:

Die Zellinie NCI-H 727 wurde mit einer Konzentration von 5 x $10^4$ Zellen/ml in 50 ml Kulturflaschen ausgebracht. Die Zellen wurden in RPM1 1640 Medium unter Zusatz von L-Glutamin (2mM), foetalem Rinderserum (10V/V) und Gentamycinsulfat (50 $\mu$g/ml) bei 7 % $CO_2$/93 % Luft gehalten. Einen Tag nach dem Ausbringen der Zellen wurde die zu untersuchende Verbindung zugesetzt. Die die Testverbindungen enthaltenden Kulturmedien wurden jeweils am dritten Tag durch frische Kulturmedien ersetzt. Die Untersuchungsergebnisse sind in Tabelle 1 angegeben.

Tabelle 1

| Dosis – Wirkungsbeziehung des tumorwachstumshemmenden Effektes von 1 in der von menschlichen Lungentumoren abgeleiteten Zellinie NCI – H 727 | | | | |
|---|---|---|---|---|
| Behandlung | Zahl der lebensfähigen Zellen X $10^4$/ml | | | |
| | Ausbringung | 72 Std. | 144 Std. | 216 Std. |
| Kontrolle | 5 | 5,7 | 8,9 | 38,3 |
| Polyethylenglycol – Kontrolle | 5 | 7,5 | 28,7 | 72,8 |
| 1 1,0 µM | 5 | 0 | 0 | 0 |
| 1 0,5 µM | 5 | 1,2 | 1,3 | 1,6 |
| 1 0,1 µM | 5 | 1,7 | 1,9 | 2,8 |

2. Einfluß von 1 auf die Wachstumsgeschwindigkeit der von menschlichen Adenocarcinom – Lungentumoren abgeleiteten Zellinie NCI – H 322 (Clara cell)

Methoden und Ergebnisse:

Die Zellinie NCI – H 322 wurde mit einer Konzentration von 5 x $10^4$ Zellen/ml in 50 ml Kulturflaschen ausgebracht. Die Zellen wurden in RPM1 1640 Medium unter Zusatz von L – Glutamin (2mM), foetalem Rinderserum (10V/V) und Gentamycinsulfat (50 µg/ml) bei 7 % $CO_2$/93 % Luft gehalten. Einen Tag nach dem Ausbringen der Zellen wurde die zu untersuchende Verbindung zugesetzt. Nach dem Anfärben mit Trypanblau nach den in Tabelle 2 angegebenen Zeitintervallen wurden die lebensfähigen Zellen gezählt. Die Ergebnisse sind in Tabelle 2 angegeben.

Tabelle 2

| Dosis – Wirkungsbeziehung des tumorwachstumshemmenden Effektes von 1 in der von menschlichen Adenocarcinom – Lungentumoren abgeleiteten Zellinie NCI – H 322 (Clara cell) | | | | |
|---|---|---|---|---|
| Behandlung | Zahl der lebensfähigen Zellen X $10^4$/ml | | | |
| | Ausbringung | 72 Std. | 144 Std. | 216 Std. |
| Kontrolle | 5 | 5,5 | 10,4 | 33,6 |
| Polyethylenglycol – Kontrolle | 5 | 7,5 | 22,7 | 34,3 |
| 1 1,0 µM | 5 | 0 | 0 | 0 |
| 1 0,5 µM | 5 | 0,7 | 0,9 | 1,9 |
| 1 0,1 µM | 5 | 1,1 | 1,4 | 2,0 |

3. Einfluß von 1 auf die Wachstumsgeschwindigkeit der von menschlichen Adenocarcinom – Lungentumoren abgeleiteten Zellinie NCI – H 358 (alveolar type II cell)

Methoden und Ergebnisse:

Die Zellinie NCI – H 358 wurde mit einer Konzentration von 5 x $10^4$ Zellen/ml in 50 ml Kulturflaschen ausgebracht. Die Zellen wurden in RPM1 1640 Medium unter Zusatz von L – Glutamin (2mM), foetalem Rinderserum (10V/V) und Gentamycinsulfat (50 µg/ml) bei 7 % $CO_2$/93 % Luft gehalten. Einen Tag nach dem Ausbringen der Zellen wurde die zu untersuchende Verbindung zugesetzt. Nach dem Anfärben mit Trypanblau nach den in Tabelle 3 angegebenen Zeitintervallen wurden die lebensfähigen Zellen gezählt. Die Ergebnisse sind in Tabelle 3 angegeben.

Tabelle 3

| Dosis – Wirkungsbeziehung des tumorwachstumshemmenden Effektes von 1 in der von menschlichen Adenocarcinom – Lungentumoren abgeleiteten Zellinie NCI – H 358 (alveolar type II cell) | | | | |
|---|---|---|---|---|
| Behandlung | Zahl der lebensfähigen Zellen X $10^4$/ml | | | |
| | Ausbringung | 72 Std. | 144 Std. | 216 Std. |
| Kontrolle | 5 | 5,8 | 16,8 | 40,6 |
| Polyethylenglycol – Kontrolle | 5 | 7,6 | 25,9 | 80,6 |
| 1 1,0 µM | 5 | 0 | 0 | 0 |
| 1 0,5 µM | 5 | 0,9 | 1,1 | 1,8 |
| 1 0,1 µM | 5 | 0,9 | 1,2 | 2,0 |

## Patentansprüche

**1.** Verwendung von optisch reinen Diarylverbindungen der Formel I

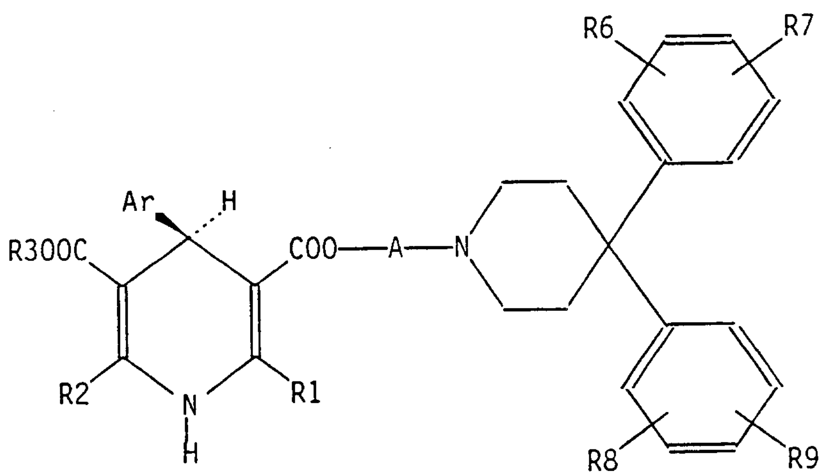

(I)

worin

Ar einen Cyclus der Formel

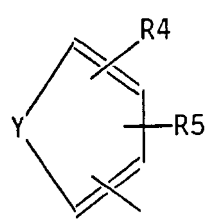

darstellt, in dem Y Sauerstoff (O), Schwefel (S), Vinylen ( – CH = CH – ), Azomethin ( – CH = N – ) oder eine Gruppe der Formel

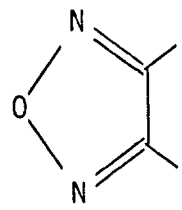

oder

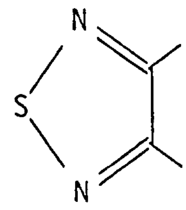

bedeutet,

R1 Wasserstoff, 1 – 6C – Alkyl oder 3 – 7C – Alkoxyalkyl bedeutet,

R2 Wasserstoff, Amino ($NH_2$), 1 – 6C – Alkyl oder 3 – 7C – Alkoxyalkyl bedeutet,

R3 Wasserstoff, 1 – 6C – Alkyl oder 3 – 7C – Alkoxyalkyl bedeutet,

R4 und R5 gleich oder verschieden sind und Wasserstoff, Hydroxy, Halogen, Nitro, Cyano, Trifluormethyl, 1 – 4C – Alkyl, 1 – 4C – Alkoxy, ganz oder teilweise durch Fluor substituiertes 1 – 4C – Alkoxy, 1 – 4C – Alkoxycarbonyl, 2 – 5C – Acyl, Amino, Mono – oder Di – 1 – 4C – alkylamino oder gemeinsam Methylendioxy bedeuten,

R6, R7, R8 und R9 gleich oder verschieden sind und Wasserstoff, Hydroxy, Halogen, 1 – 4C – Alkyl, 1 – 4C – Alkoxy oder ganz oder teilweise durch Fluor substituiertes 1 – 4C – Alkoxy bedeuten,

A 2 – 5C – Alkylen oder A1 – O – A2 bedeutet,
wobei
A1 2 – 4C – Alkylen und
A2 2 – 4C – Alkylen oder 2C – Alkylenoxy – 2C – alkylen bedeutet,

und ihren Salzen zur Herstellung von Arzneimitteln für die Behandlung von Tumorerkrankungen.

2. Verwendung gemäß Anspruch 1 von Verbindungen der Formel I nach Anspruch 1, worin Ar 3 – Nitrophenyl, 2 – Chlorphenyl, 2,3 – Dichlorphenyl, 2 – Trifluormethylphenyl, 2 – Difluormethoxyphenyl, 2,3 – Methylendioxyphenyl oder 2,1,3 – Benzoxdiazol – 4 – yl, R1 Methyl, R2 Methyl, R3 Methyl, Ethyl oder Methoxyethyl, R6, R7, R8 und R9 Wasserstoff und A Ethylen oder Propylen bedeutet, und ihren Salzen.

3. Verwendung gemäß Anspruch 1 von Verbindungen der Formel I nach Anspruch 1, worin Ar 3 – Nitrophenyl, 2 – Chlorphenyl, 2,3 – Dichlorphenyl, 2 – Trifluormethylphenyl, 2 – Difluormethoxyphenyl, 2,3 – Methylendioxyphenyl oder 2,1,3 – Benzoxdiazol – 4 – yl, R1 Methyl, R2 Amino, R3 Methyl, Ethyl oder Methoxyethyl, R6, R7, R8 und R9 Wasserstoff und A Ethylen oder Propylen bedeutet, und ihren Salzen.

4. Verwendung gemäß Anspruch 1 von Verbindungen der Formel I nach Anspruch 1, worin Ar 3 – Nitrophenyl, 2 – Chlorphenyl, 2,3 – Dichlorphenyl, 2 – Trifluormethylphenyl, 2 – Difluormethoxyphenyl, 2,3 – Methylendioxyphenyl oder 2,1,3 – Benzoxdiazol – 4 – yl, R1 Methyl, R2 Methyl, R3 Methyl, Ethyl oder Methoxyethyl, R6, R7, R8 und R9 Wasserstoff und A A1 – O – A2 bedeutet, wobei A1 Ethylen und A2 Ethylen oder Ethylenoxyethylen bedeutet, und ihren Salzen.

5. Verwendung gemäß Anspruch 1 von Verbindungen der Formel I nach Anspruch 1, worin Ar 3 – Nitrophenyl oder 2,3 – Dichlorphenyl, R1 Methyl, R2 Methyl, R3 Methyl oder Ethyl, R6, R7, R8 und R9 Wasserstoff und A Ethylen oder Propylen bedeutet, und ihren Salzen.

6. Verwendung gemäß Anspruch 1 von Verbindungen der Formel I nach Anspruch 1, worin Ar 3 – Nitrophenyl oder 2,3 – Dichlorphenyl, R1 Methyl, R2 Amino, R3 Methyl oder Ethyl, R6, R7, R8 und R9 Wasserstoff und A Ethylen oder Propylen bedeutet, und ihren Salzen.

7. Verwendung gemaß Anspruch 1 von Verbindungen der Formel I nach Anspruch 1, worin Ar 3 – Nitrophenyl oder 2,3 – Dichlorphenyl, R1 Methy, R2 Methyl, R3 Methyl oder Ethyl, R6, R7, R8 und R9 Wasserstoff und A A1 – O – A2 bedeutet, wobei A1 Ethylen und A2 Ethylen bedeutet, und ihren Salzen.

**8.** Verwendung der Verbindung (R)-(+)-1,4-Dihydro-2,6-dimethyl-4-(3-nitrophenyl)-pyridin-3,5-dicarbonsäure-3-methyl-5-[3-(4,4-diphenyl-1-piperidinyl)-propyl]-ester, und ihrer Salze zur Herstellung von Arzneimitteln für die Behandlung von Tumorerkrankungen.

## Claims

**1.** Use of optically pure diaryl compounds of formula I

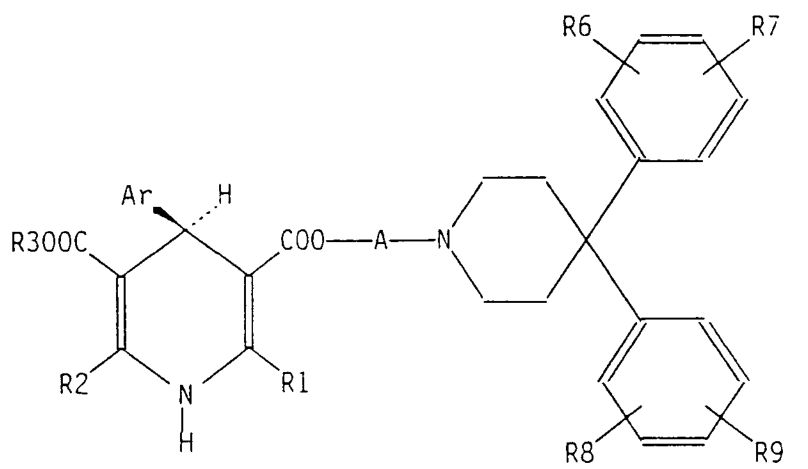

(I)

wherein

Ar represents a ring of the formula

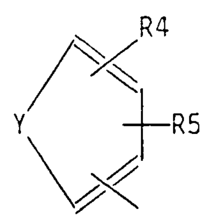

in which Y denotes oxygen (O), sulphur (S), vinylene (-CH=CH-), azomethine (-CH=N-) or a group of the formula

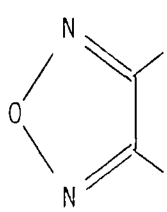

or

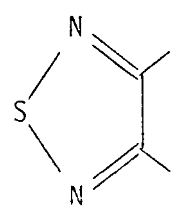

R1 denotes hydrogen, 1-6C-alkyl or 3-7C-alkoxyalkyl,

R2 denotes hydrogen, amino ($NH_2$), 1-6C-alkyl or 3-7C-alkoxyalkyl,

R3 denotes hydrogen, 1-6C-alkyl or 3-7C-alkoxyalkyl,

R4 and R5 are identical or different and denote hydrogen, hydroxyl, halogen, nitro, cyano, trifluormethyl, 1-4C-alkyl, 1-4C-alkoxy, 1-4C-alkoxy which is completely or partly substituted by fluorine, 1-4C-alkoxycarbonyl, 2-5C-acyl, amino, mono- or di-1-4C-alkylamino or together methylenedioxy,

R6, R7, R8 and R9 are identical or different and denote hydrogen, hydroxyl, halogen, 1-4C-alkyl, 1-4C-alkoxy or 1-4C-alkoxy which is completely or partly substituted by fluorine, and

A denotes 2 – 5C – alkylene or A1 – O – A2,
in which
A1 denotes 2 – 4C – alkylene and
A2 denotes 2 – 4C – alkylene or 2C – alkyleneoxy – 2C – alkylene

and their salts for the manufacture of medicaments for the treatment of tumors.

2. Use, according to claim 1, of compounds of formula I, according to claim 1, wherein Ar denotes 3 – nitrophenyl, 2 – chlorophenyl, 2,3 – dichlorophenyl, 2 – trifluoromethylphenyl, 2 – difluoromethoxyphenyl, 2,3 – methylenedioxyphenyl or 2,1,3 – benzoxdiazol – 4 – yl, R1 denotes methyl, R2 denotes methyl, R3 denotes methyl, ethyl or methoxyethyl, R6, R7, R8 and R9 denote hydrogen and A denotes ethylene or propylene, and of their salts.

3. Use, according to claim 1, of compounds of formula I, according to claim 1, wherein Ar denotes 3 – nitrophenyl, 2 – chlorophenyl, 2,3 – dichlorophenyl, 2 – trifluoromethylphenyl, 2 – difluoromethoxyphenyl, 2,3 – methylenedioxyphenyl or 2,1,3 – benzoxdiazol – 4 – yl, R1 denotes methyl, R2 denotes amino, R3 denotes methyl, ethyl or methoxyethyl, R6, R7, R8 and R9 denote hydrogen and A denotes ethylene or propylene, and of their salts.

4. Use, according to claim 1, of compounds of formula I, according to claim 1, wherein Ar denotes 3 – nitrophenyl, 2 – chlorophenyl, 2,3 – dichlorophenyl, 2 – trifluoromethylphenyl, 2 – difluoromethoxyphenyl, 2,3 – methylenedioxyphenyl or 2,1,3 – benzoxdiazol – 4 – yl, R1 denotes methyl, R2 denotes methyl, R3 denotes methyl, ethyl or methoxyethyl, R6, R7, R8 and R9 denote hydrogen and A denotes A1 – O – A2, A1 being ethylene and A2 being ethylene or ethyleneoxyethylene, and of their salts.

5. Use, according to claim 1, of compounds of formula I, according to claim 1, wherein Ar denotes 3 – nitrophenyl or 2,3 – dichlorophenyl, R1 denotes methyl, R2 denotes methyl, R3 denotes methyl or ethyl, R6, R7, R8 and R9 denote hydrogen and A denotes ethylene or propylene, and of their salts.

6. Use, according to claim 1, of compounds of formula I, according to claim 1, wherein Ar denotes 3 – nitrophenyl or 2,3 – dichlorophenyl, R1 denotes methyl, R2 denotes amino, R3 denotes methyl or ethyl, R6, R7, R8 and R9 denote hydrogen and A denotes ethylene or propylene, and of their salts.

7. Use, according to claim 1, of compounds of formula I, according to claim 1, wherein Ar denotes 3 – nitrophenyl or 2,3 – dichlorophenyl, R1 denotes methyl, R2 denotes methyl, R3 denotes methyl or ethyl, R6, R7, R8 and R9 denote hydrogen and A denotes A1 – O – A2, A1 being ethylene and A2 being ethylene, and of their salts.

8. Use of the compound (R) – ( + ) – 3 – methyl – 5 – [3(4,4 – diphenyl – 1 – piperidinyl) – propyl] – 1,4 – dihydro – 2,6 – dimethyl – 4 – (3 – nitrophenyl) – pyridine – 3,5 – dicarboxylate and of its salts for the manufacture of medicaments for the treatment of tumors.

## Revendications

**1.** Utilisation de composés diaryliques optiquement purs de formule I

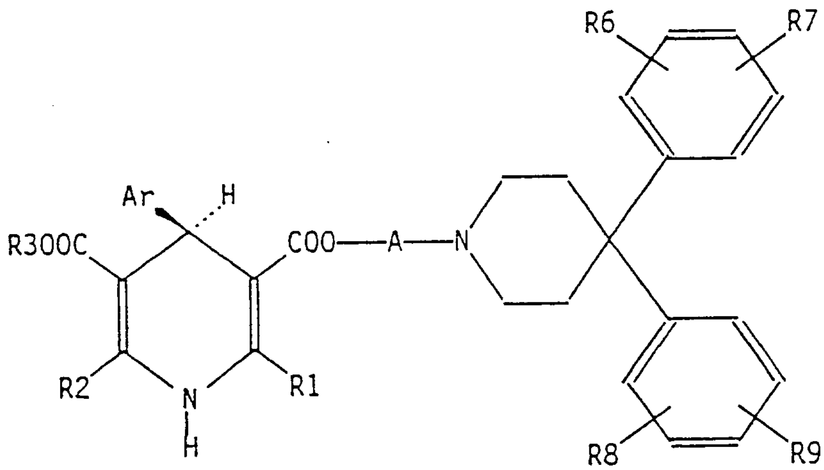

(I)

dans laquelle
Ar représente un cycle de formule

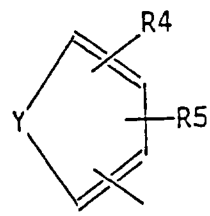

dans laquelle Y représente l'oxygène (O), le soufre (S), le groupe vinylène ( $-CH=CH-$ ), le groupe azométhine ( $-CH=N-$ ) ou un groupe de formule

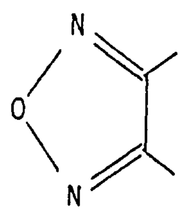

ou

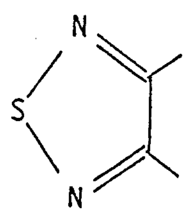

R1 représente un atome d'hydrogène, un groupe alkyle en $C_1-C_6$ ou un groupe alcoxyalkyle en $C_3-C_7$.
R2 représente un atome d'hydrogène, un groupe amino ($NH_2$), un groupe alkyle en $C_1-C_6$ ou un groupe alcoxyalkyle en $C_3-C_7$,
R3 représente un atome d'nydrogène, un groupe alkyle en $C_1-C_6$ ou un groupe alcoxyalkyle en $C_3-C_7$,
R4 et R5 sont identiques ou différents et représentent un atome d'hydrogène, un groupe hydroxyle, un atome d'halogène, un groupe nitro, un groupe cyano, un groupe trifluorométhyle, un groupe alkyle en $C_1-C_4$, un groupe alcoxy en $C_1-C_4$,
un groupe alcoxy en $C_1-C_4$ totalement ou partiellement substitué par le fluor, un groupe alcoxycarbonyle en $C_1-C_4$, un groupe acyle en $C_2-C_5$, un groupe amino, un groupe mono- ou dialkylamino

en $C_1-C_4$ ou, ensemble le groupement méthylènedioxy,
R6, R7, R8 et R9 sont identiques ou différents et représentent un atome d'hydrogène, un groupe hydroxyle, un atome d'halogène, un groupe alkyle en $C_1-C_4$, un groupe alcoxy en $C_1-C_4$ ou un groupe alcoxy en $C_1-C_4$ totalement ou partiellement substitué par le fluor,
A représente un groupe alkylène en $C_2-C_5$ ou A1 – O – A2,
où
A1 représente un groupe alkylène en $C_2-C_4$ et
A2 représente un groupe alkylène en $C_2-C_4$ ou un groupe alkylèneoxy en $C_2$ – alkylène en $C_2$,
et leurs sels pour la préparation de médicaments pour le traitement de maladies tumorales.

2. Utilisation selon la revendication 1 de composés de formule I selon la revendication 1, dans laquelle Ar représente un groupe parmi les suivants 3 – nitrophényle, 2 – chlorophényle, 2,3 – dichlorophényle, 2 – trifluorométhylphényle, 2 – difluorométhoxyphényle, 2,3 – méthylènedioxyphényle ou 2,1,3 – benzoxadiazol – 4 – yle, R1 représente le groupe méthyle, R2 représente le groupe méthyle, R3 représente le groupe méthyle, le groupe éthyle ou le groupe méthoxyéthyle, R6, R7, R8 et R9 représentent un atome d'hydrogène et A représente le groupe éthylène ou le groupe propylène, et leurs sels.

3. Utilisation selon la revendication 1 de composé de formule I selon la revendication 1, dans laquelle Ar représente un groupe parmi les suivants : 3 – nitro – phényle, 2 – chlorophényle, 2,3 – dichlorophényle, 2 – trifluorométhylphényle, 2 – difluorométhoxyphényle, 2,3 – méthylènedioxyphényle ou 2,1,3 – benzoxadiazol – 4 – yle, R1 représente le groupe méthyle, R2 représente un groupe amino, R3 repré – sente le groupe méthyle, le groupe éthyle ou le groupe méthoxyéthyle, R6, R7, R8 et R9 représentent l'hydrogène et A représente le groupe éthylène ou le groupe propylène, et leurs sels.

4. Utilisation selon la revendication 1 de composés de formule I selon la revendication 1, dans laquelle Ar représente un groupe parmi les suivants 3 – nitrophényle, 2 – chorophényle, 2,3 – dichlorophényle, 2 – trifluorométhylphényle, 2 – difluorométhoxyphényle, 2,3 – méthylènedioxyphényle ou 2,1,3 – benzoxadiazol – 4 – yle, R1 représente le groupe méthyle, R2 représente le groupe méthyle, R3 représente le groupe méthyle, le groupe éthyle ou le groupe méthoxyéthyle, R6, R7, R8 et R9 représentent l'hydrogène et A représente A1 – O – A2 où A1 représente le groupe éthylène et A2 représente le groupe éthylène ou éthylèneoxyéthylène, et leurs sels.

5. Utilisation selon la revendication 1 de composés de formule I selon la revendication 1, dans laquelle A représente le groupe 3 – nitrophényle ou le groupe 2,3 – dichlorophényle, R1 représente le groupe méthyle, R2 représente le groupe méthyle, R3 représente le groupe méthyle ou le groupe éthyle, R6, R7, R8 et R9 représentent l'hydrogène et A représente le groupe éthylène ou le groupe propylène, et leurs sels.

6. Utilisation selon la revendication 1 de composés de formule I selon la revendication 1, dans laquelle A représente le groupe 3 – nitrophényle ou le groupe 2,3 – dichlorophényle, R1 représente le groupe méthyle, R2 représente un groupe amino, R3 représente le groupe méthyle ou le groupe éthyle, R6, R7, R8 et R9 représentent l'hydrogène et A représente le groupe éthylène ou le groupe propylène, et leurs sels.

7. Utilisation selon la revendication 1 de composés de formule I selon la revendication 1, dans laquelle A représente le groupe 3 – nitrophényle ou le groupe 2,3 – dichlorophényle, R1 représente le groupe méthyle, R2 représente le groupe méthyle, R3 représente le groupe méthyle ou le groupe éthyle, R6, R7, R8 et R9 représentent l'hydrogène et A représente A1 – O – A2 où A1 représente l'éthylène et A2 représente l'éthylène, et leurs sels.

8. Utilisation du composé (R) – ( + ) – 1,4 – dihydro – 2,6 – diméthyl – 4 – (3 – nitrophényl) – pyridin – 3,5 – dicarboxylate de 3 – méthyle et de 5 – [3 – (4,4 – diphényl – 1 – pipéridinyl) – propyle] et ses sels pour la préparation de médicaments pour le traitement de maladies tumorales.